# EUROPEAN PATENT SPECIFICATION

(11) **EP 1 903 109 B1**
(45) Date of publication and mention of the grant of the patent: **10.03.2010**
(21) Application number: 06019930.4
(22) Date of filing: 22.09.2006
(51) Int. Cl.: C12N 9/14

(54) **Human protein phosphatase with EF-hands-1(PPEF-1)-related gene variant associated with T-cell lymphoblastic lymphoma**
Menschliche protein phosphatase mit EF-hände-1 (PPEF-1)- ähnliches genvariant assoziert mit T-zellen lymphoblastisches Lymphom
Variant du gène proteine-phosphatase humaine avec mains EF (PPEF-1) associée au lymphome lymphoblastique de cellules de T

(43) Date of publication of application: 26.03.2008
(73) Proprietor: Visgeneer, Inc., Hsinchu City 300 (TW)
(72) Inventor: Dai, Ken-Shwo, Hsinchu City 300 (TW)
(74) Representative: Epping - Hermann - Fischer

(56) References cited:
- WO-A2-2004/070383
- MONTINI E ET AL: "A novel human serine-threonine phosphatase related to the Drosophila retinal degeneration C (rdgC) gene is selectively expressed in sensory neurons of neural crest origin" HUMAN MOLECULAR GENETICS, OXFORD UNIVERSITY PRESS, SURREY, GB, vol. 6, no. 7, 1997, pages 1137-1145, XP002304435 ISSN: 0964-6906
- SHERMAN P M ET AL: "Identification and characterization of a conserved family of protein serine/threonine phosphatases homologous to Drosophila retinal degeneration C (rdgC)" PROCEEDINGS OF THE NATIONAL ACADEMY OF SCIENCES OF USA, NATIONAL ACADEMY OF SCIENCE, WASHINGTON, DC, US, vol. 94, no. 21, October 1997 (1997-10), pages 11639-11644, XP002304436 ISSN: 0027-8424
- HUANG X ET AL: "Molecular cloning, expression, and characterization of a novel human serine/threonine protein phosphatase, PP7, that is homologous to Drosophila retinal degeneration C gene product (rdgC)" JOURNAL OF BIOLOGICAL CHEMISTRY, AMERICAN SOCIETY OF BIOLOCHEMICAL BIOLOGISTS, BIRMINGHAM,, US, vol. 273, no. 3, 16 January 1998 (1998-01-16), pages 1462-1468, XP002304437 ISSN: 0021-9258
- KUTUZOV M A ET AL: "Expression and characterization of PP7, a novel plant protein Ser/Thr phosphatase distantly related to RdgC/PPEF and PP5" FEBS LETTERS, ELSEVIER, AMSTERDAM, NL, vol. 440, no. 1-2, 27 November 1998 (1998-11-27), pages 147-152, XP004463517 ISSN: 0014-5793
- HUOPANIEMI LAURA ET AL: "Characterization of two unusual RS1 gene deletions segregating in Danish retinoschisis families" HUMAN MUTATION, vol. 16, no. 4, 2000, pages 307-314, XP002420624 ISSN: 1059-7794

### SEQUENCE LISTING

<110> visgeneer Inc.
<120> HUMAN PROTEIN PHOSPHATASE WITH EF-HANDS-1 (PPEF-1)-RELATED GENE VARIANT ASSOCIATED WITH T-CELL LYMPHOBLASTIC LYMPHOMA
<130> none
<160> 4
<170> PatentIn version 3.3
<210> 1
   <211> 2480
   <212> DNA
   <213> Homo sapiens
<400> 1
   agcttaaagg gaggcacttt tcacactctg tcttaaaatc agaagaagaa ttcatgaaca      60
   catatgattt agatagaagt catgggatgc agcagttctt caacgaaaac caggagatct     120
   gacacatcac tgagagctgc gttgatcatc cagaactggt accgaggtta caaagctcga     180
   ctgaaggcca gacaacacta tgccctcacc atcttccagt ccatcgaata tgctgatgaa     240
   caaggccaaa tgcagttatc caccttcttt tccttcatgt tggaaaacta cacacatata     300
   cataaggaag agctagaatt aagaaatcag tctcttgaaa gcgaacagga catgagggat     360
   agatgggatt atgtggactc gatagatgtc ccagactcct ataatggtcc tcggctacaa     420
   tttcctctca cttgtacgga tattgattta cttcttgagg ccttcaagga acaacagata     480
   cttcatgccc attatgtctt agaggtgcta tttgaaacca agaaagtcct gaagcaaatg     540
   ccgaatttca ctcacataca aacttctccc tccaaagagg taacaatctg tggtgatttg     600
   catgggaaac tggatgatct ttttttgatc ttctacaaga atggtctccc ctcagagagg      660
   aacccgtatg tttttaatgg tgactttgta gatcgaggaa agaattccat agagatccta     720
   atgatcctgt gtgtgagttt tcttgtctac cccaatgacc tgcacttgaa cagagggaac     780
   cacgaagatt ttatgatgaa tctgaggtat ggcttcacga aagaaatttt gcataaatat     840
   aagctacatg gaaaaagaat cttacaaatc ttggaagaat tctatgcctg gctcccaatc     900
   ggtacaatcg ttgacaatga aatcctggtc atccatggtg ggatatcaga gaccacagac     960
   ttgaatttac tccaccgtgt agagaggaac aagatgaaat ctgtgctgat accaccaacg    1020
   gaaacaaaca gagaccatga cactgactcg aagcacaata aagtaggtgt gacttttaat    1080
   gcacatggaa gaatcaaaac aaatggatct cctactgaac acttaacaga gcatgaatgg    1140
   gaacagatta ttgatattct gtggagtgat cccagaggca aaaatggctg ttttccaaat    1200
   acgtgccgag gagggggctg ctattttgga ccagatgtta cttccaagat tcttaataaa   1260
   taccagttga agatgctcat caggtctcat gaatgtaagc ccgaagggta tgaaatctgt    1320
   catgatggga aggtggtgac tatattttct gcttctaatt attatgaaga aggcagcaat    1380
   cgaggagctt acatcaaact atgttctggt acaactcctc gatttttcca gtaccaagta    1440
   actaaagcaa cgtgctttca gcctcttcgc caaagagtgg atactatgga aaacagcgcc     1500
   atcaagatat taagagagag agtgatttca cgaaaaagtg accttactcg tgctttccaa    1560
   cttcaagacc acagaaaatc aggaaaactt tctgtgagcc agtgggcttt ttgcatggag    1620
   aacattttgg ggctgaactt accatggaga tccctcagtt cgaatctggt aaacatagac    1680
   caaaatggaa acgttgaata catgtccagc ttccagaata tccgcattga aaaacctgta    1740
   caagaggctc attctactct agttgaaact ctgtacagat acagatctga cctggaaatc    1800
   atatttaatg ccattgacac tgatcactca ggcctgatct ccgtggaaga atttcgtgcc    1860
   atgtggaaac tttttagttc tcactacaat gttcacattg atgattccca agtcaataag    1920
   cttgccaaca taatggactt gaacaaagat ggaagcattg actttaatga gtttttaaag    1980
   gctttctatg tagtgcatag atatgaagac ttgatgaaac ctgatgtcac caaccttggc    2040
   taaacacaaa tgagagcttc cctcaggctc cctgaaacag ctaggcccaa atcacaagta    2100
   cagtcctttc caacacccct gaaattcata gtcagtagca gagaaaagca gatcccaatt    2160
   catcccacaa acagatgcat agtatgggtt ttggaagtcc ctagcaagct gttattggta    2220
   agattaggtt aaatgtcagt aataggattt ggtttcagca ttagtaccta catattgcca    2280
   gtgagaaact gggttggacc tagtggtgtt gtcgtgagtg ccacctaacc aggaggccag    2340
   agcggtttga aaacatcctg aaaggaactc atacagcaca agagaaaact actaagcttg    2400
   acatctgtga gtgactgagg gagacaggag gaataccagg ttattcatgg aataaagtct    2460
   ttccatcttt aaaaaaaaaa                                                2480
<210> 2
   <211> 653
   <212> PRT
   <213> Homo sapiens
<400> 2
   Met Gly Cys Ser Ser Ser Ser Thr Lys Thr Arg Arg Ser Asp Thr Ser
   1 5 10 15
   Leu Arg Ala Ala Leu Ile Ile Gln Asn Trp Tyr Arg Gly Tyr Lys Ala
   20 25 30
   Arg Leu Lys Ala Arg Gln His Tyr Ala Leu Thr Ile Phe Gln Ser Ile
   35 40 45
   Glu Tyr Ala Asp Glu Gln Gly Gln Met Gln Leu Ser Thr Phe Phe Ser
   50 55 60
   Phe Met Leu Glu Asn Tyr Thr His Ile His Lys Glu Glu Leu Glu Leu
   65 70 75 80
   Arg Asn Gln Ser Leu Glu Ser Glu Gln Asp Met Arg Asp Arg Trp Asp
   85 90 95
   Tyr Val Asp Ser Ile Asp Val Pro Asp Ser Tyr Asn Gly Pro Arg Leu
                   100                 105                 110
   Gln Phe Pro Leu Thr Cys Thr Asp Ile Asp Leu Leu Leu Glu Ala Phe
           115                 120                 125
   Lys Glu Gln Gln Ile Leu His Ala His Tyr Val Leu Glu Val Leu Phe
       130                 135                 140
   Glu Thr Lys Lys Val Leu Lys Gln Met Pro Asn Phe Thr His Ile Gln
   145                 150                 155                 160
   Thr Ser Pro Ser Lys Glu Val Thr Ile Cys Gly Asp Leu His Gly Lys
                   165                 170                 175
   Leu Asp Asp Leu Phe Leu Ile Phe Tyr Lys Asn Gly Leu Pro Ser Glu
               180                 185                 190
   Arg Asn Pro Tyr Val Phe Asn Gly Asp Phe Val Asp Arg Gly Lys Asn
           195                 200                 205
   Ser Ile Glu Ile Leu Met Ile Leu Cys Val Ser Phe Leu Val Tyr Pro
       210                 215                 220
   Asn Asp Leu His Leu Asn Arg Gly Asn His Glu Asp Phe Met Met Asn
   225                 230                 235                 240
   Leu Arg Tyr Gly Phe Thr Lys Glu Ile Leu His Lys Tyr Lys Leu His
                   245                 250                 255
   Gly Lys Arg Ile Leu Gln Ile Leu Glu Glu Phe Tyr Ala Trp Leu Pro
               260                 265                 270
   Ile Gly Thr Ile Val Asp Asn Glu Ile Leu Val Ile His Gly Gly Ile
           275                 280                 285
   Ser Glu Thr Thr Asp Leu Asn Leu Leu His Arg Val Glu Arg Asn Lys
       290                 295                 300
   Met Lys Ser Val Leu Ile Pro Pro Thr Glu Thr Asn Arg Asp His Asp
   305                 310                 315                 320
   Thr Asp Ser Lys His Asn Lys Val Gly Val Thr Phe Asn Ala His Gly
                   325                 330                 335
   Arg Ile Lys Thr Asn Gly Ser Pro Thr Glu His Leu Thr Glu His Glu
               340                 345                 350
   Trp Glu Gln Ile Ile Asp Ile Leu Trp Ser Asp Pro Arg Gly Lys Asn
           355                 360                 365
   Gly Cys Phe Pro Asn Thr Cys Arg Gly Gly Gly Cys Tyr Phe Gly Pro
   370 375 380
   Asp Val Thr Ser Lys Ile Leu Asn Lys Tyr Gln Leu Lys Met Leu Ile
   385 390 395 400
   Arg Ser His Glu Cys Lys Pro Glu Gly Tyr Glu Ile Cys His Asp Gly
   405 410 415
   Lys Val Val Thr Ile Phe Ser Ala Ser Asn Tyr Tyr Glu Glu Gly Ser
   420 425 430
   Asn Arg Gly Ala Tyr Ile Lys Leu Cys Ser Gly Thr Thr Pro Arg Phe
   435 440 445
   Phe Gln Tyr Gln Val Thr Lys Ala Thr Cys Phe Gln Pro Leu Arg Gln
   450 455 460
   Arg Val Asp Thr Met Glu Asn Ser Ala Ile Lys Ile Leu Arg Glu Arg
   465 470 475 480
   Val Ile Ser Arg Lys Ser Asp Leu Thr Arg Ala Phe Gln Leu Gln Asp
   485 490 495
   His Arg Lys Ser Gly Lys Leu Ser Val Ser Gln Trp Ala Phe Cys Met
   500 505 510
   Glu Asn Ile Leu Gly Leu Asn Leu Pro Trp Arg Ser Leu Ser Ser Asn
   515 520 525
   Leu Val Asn Ile Asp Gln Asn Gly Asn Val Glu Tyr Met Ser Ser Phe
   530 535 540
   Gln Asn Ile Arg Ile Glu Lys Pro Val Gln Glu Ala His Ser Thr Leu
   545 550 555 560
   Val Glu Thr Leu Tyr Arg Tyr Arg Ser Asp Leu Glu Ile Ile Phe Asn
   565 570 575
   Ala Ile Asp Thr Asp His Ser Gly Leu Ile Ser Val Glu Glu Phe Arg
   580 585 590
   Ala Met Trp Lys Leu Phe Ser Ser His Tyr Asn Val His Ile Asp Asp
   595 600 605
   Ser Gln Val Asn Lys Leu Ala Asn Ile Met Asp Leu Asn Lys Asp Gly
   610 615 620
   Ser Ile Asp Phe Asn Glu Phe Leu Lys Ala Phe Tyr Val Val His Arg
   625 630 635 640
   Tyr Glu Asp Leu Met Lys Pro Asp Val Thr Asn Leu Gly
   645 650
<210> 3
   <211> 2130
   <212> DNA
   <213> Homo sapiens
<400> 3
   agcttaaagg gaggcacttt tcacactctg tcttaaaatc agaagaagaa ttcatgaaca    60
   catatgattt agatagaagt catgggatgc agcagttctt caacgaaaac caggagatct   120
   gacacatata cttcatgccc attatgtctt agaggtgcta tttgaaacca agaaagtcct   180
   gaagcaaatg ccgaatttca ctcacataca aacttctccc tccaaagagg taacaatctg   240
   tggtgatttg catgggaaac tggatgatct ttttttgatc ttctacaaga atggtctccc   300
   ctcagagagg aacccgtatg tttttaatgg tgactttgta gatcgaggaa agaattccat   360
   agagatccta atgatcctgt gtgtgagttt tcttgtctac cccaatgacc tgcacttgaa   420
   cagagggaac cacgaagatt ttatgatgaa tctgaggtat ggcttcacga aagaaatttt   480
   gcataaatat aagctacatg gaaaaagaat cttacaaatc ttggaagaat tctatgcctg   540
   gctcccaatc ggtacaatcg ttgacaatga aatcctggtc atccatggtg ggatatcaga   600
   gaccacagac ttgaatttac tccaccgtgt agagaggaac aagatgaaat ctgtgctgat   660
   accaccaacg gaaacaaaca gagaccatga cactgactcg aagcacaata aagtaggtgt   720
   gacttttaat gcacatggaa gaatcaaaac aaatggatct cctactgaac acttaacaga   780
   gcatgaatgg gaacagatta ttgatattct gtggagtgat cccagaggca aaaatggctg   840
   ttttccaaat acgtgccgag gagggggctg ctattttgga ccagatgtta cttccaagat   900
   tcttaataaa taccagttga agatgctcat caggtctcat gaatgtaagc ccgaagggta   960
   tgaaatctgt catgatggga aggtggtgac tatattttct gcttctaatt attatgaaga  1020
   aggcagcaat cgaggagctt acatcaaact atgttctggt acaactcctc gatttttcca  1080
   gtaccaagta actaaagcaa cgtgctttca gcctcttcgc caaagagtgg atactatgga  1140
   aaacagcgcc atcaagatat taagagagag agtgatttca cgaaaaagtg accttactcg  1200
   tgctttccaa cttcaagacc acagaaaatc aggaaaactt tctgtgagcc agtgggcttt  1260
   ttgcatggag aacattttgg ggctgaactt accatggaga tccctcagtt cgaatctggt  1320
   aaacatagac caaaatggaa acgttgaata catgtccagc ttccagaata tccgcattga  1380
   aaaacctgta caagaggctc attctactct agttgaaact ctgtacagat acagatctga  1440
   cctggaaatc atatttaatg ccattgacac tgatcactca ggcctgatct ccgtggaaga  1500
   atttcgtgcc atgtggaaac tttttagttc tcactacaat gttcacattg atgattccca  1560
   agtcaataag cttgccaaca taatggactt gaacaaagat ggaagcattg actttaatga  1620
   gtttttaaag gctttctatg tagtgcatag atatgaagac ttgatgaaac ctgatgtcac  1680
   caaccttggc taaacacaaa tgagagcttc cctcaggctc cctgaaacag ctaggcccaa  1740
   atcacaagta cagtcctttc caacacccct gaaattcata gtcagtagca gagaaaagca  1800
   gatcccaatt catcccacaa acagatgcat agtatgggtt ttggaagtcc ctagcaagct   1860
   gttattggta agattaggtt aaatgtcagt aataggattt ggtttcagca ttagtaccta   1920
   catattgcca gtgagaaact gggttggacc tagtggtgtt gtcgtgagtg ccacctaacc   1980
   aggaggccag agcggtttga aaacatcctg aaaggaactc atacagcaca agagaaaact   2040
   actaagcttg acatctgtga gtgactgagg gagacaggag gaataccagg ttattcatgg   2100
   aataaagtct ttccatcttt aaaaaaaaaa                                    2130
<210> 4
   <211> 501
   <212> PRT
   <213> Homo sapiens
<400> 4
   Met Pro Asn Phe Thr His Ile Gln Thr Ser Pro Ser Lys Glu Val Thr
   1               5                   10                  15
   Ile Cys Gly Asp Leu His Gly Lys Leu Asp Asp Leu Phe Leu Ile Phe
               20                  25                  30
   Tyr Lys Asn Gly Leu Pro Ser Glu Arg Asn Pro Tyr Val Phe Asn Gly
           35                  40                  45
   Asp Phe Val Asp Arg Gly Lys Asn Ser Ile Glu Ile Leu Met Ile Leu
       50                  55                  60
   Cys Val Ser Phe Leu Val Tyr Pro Asn Asp Leu His Leu Asn Arg Gly
   65                  70                  75                  80
   Asn His Glu Asp Phe Met Met Asn Leu Arg Tyr Gly Phe Thr Lys Glu
                   85                  90                  95
   Ile Leu His Lys Tyr Lys Leu His Gly Lys Arg Ile Leu Gln Ile Leu
               100                 105                 110
   Glu Glu Phe Tyr Ala Trp Leu Pro Ile Gly Thr Ile Val Asp Asn Glu
           115                 120                 125
   Ile Leu Val Ile His Gly Gly Ile Ser Glu Thr Thr Asp Leu Asn Leu
       130                 135                 140
   Leu His Arg Val Glu Arg Asn Lys Met Lys Ser Val Leu Ile Pro Pro
   145                 150                 155                 160
   Thr Glu Thr Asn Arg Asp His Asp Thr Asp Ser Lys His Asn Lys Val
                   165                 170                 175
   Gly Val Thr Phe Asn Ala His Gly Arg Ile Lys Thr Asn Gly Ser Pro
               180                 185                 190
   Thr Glu His Leu Thr Glu His Glu Trp Glu Gln Ile Ile Asp Ile Leu
   195 200 205
   Trp Ser Asp Pro Arg Gly Lys Asn Gly Cys Phe Pro Asn Thr Cys Arg
   210 215 220
   Gly Gly Gly Cys Tyr Phe Gly Pro Asp Val Thr Ser Lys Ile Leu Asn
   225 230 235 240
   Lys Tyr Gln Leu Lys Met Leu Ile Arg Ser His Glu Cys Lys Pro Glu
   245 250 255
   Gly Tyr Glu Ile Cys His Asp Gly Lys Val Val Thr Ile Phe Ser Ala
   260 265 270
   Ser Asn Tyr Tyr Glu Glu Gly Ser Asn Arg Gly Ala Tyr Ile Lys Leu
   275 280 285
   Cys Ser Gly Thr Thr Pro Arg Phe Phe Gln Tyr Gln Val Thr Lys Ala
   290 295 300
   Thr Cys Phe Gln Pro Leu Arg Gln Arg Val Asp Thr Met Glu Asn Ser
   305 310 315 320
   Ala Ile Lys Ile Leu Arg Glu Arg Val Ile Ser Arg Lys Ser Asp Leu
   325 330 335
   Thr Arg Ala Phe Gln Leu Gln Asp His Arg Lys Ser Gly Lys Leu Ser
   340 345 350
   Val Ser Gln Trp Ala Phe Cys Met Glu Asn Ile Leu Gly Leu Asn Leu
   355 360 365
   Pro Trp Arg Ser Leu Ser Ser Asn Leu Val Asn Ile Asp Gln Asn Gly
   370 375 380
   Asn Val Glu Tyr Met Ser Ser Phe Gln Asn Ile Arg Ile Glu Lys Pro
   385 390 395 400
   Val Gln Glu Ala His Ser Thr Leu Val Glu Thr Leu Tyr Arg Tyr Arg
   405 410 415
   Ser Asp Leu Glu Ile Ile Phe Asn Ala Ile Asp Thr Asp His Ser Gly
   420 425 430
   Leu Ile Ser Val Glu Glu Phe Arg Ala Met Trp Lys Leu Phe Ser Ser
   435 440 445
   His Tyr Asn Val His Ile Asp Asp Ser Gln Val Asn Lys Leu Ala Asn
   450 455 460
   Ile Met Asp Leu Asn Lys Asp Gly Ser Ile Asp Phe Asn Glu Phe Leu
   465                 470                 475                 480
   Lys Ala Phe Tyr Val Val His Arg Tyr Glu Asp Leu Met Lys Pro Asp
                   485                 490                 495
   Val Thr Asn Leu Gly
               500

## Description

### FIELD OF THE INVENTION

The invention relates to the nucleic acid and polypeptide sequences of a novel human protein phosphatase with EF-hands-1 (PPEF-1)-related gene variant, preparation process thereof, and uses of the same in diagnosing T-cell lymphoma, in particular, T-cell lymphoblastic lymphoma.

### BACKGROUND OF THE INVENTION

Cancer is one of the major causes of death in the world. According to the report from the WHO Fact Sheet-Cancer (2003), cancer accounts for 7.1 million (12.6% of the global total) deaths annually and new cancer cases are expected to increase from 10 million people in 2000 to 15 million people by 2020. Lymphomas are cancers originated in the lymphatic system. The lymphomas are divided into Hodgkin lymphoma and non-Hodgkin lymphomas. In the United States, non-Hodgkin lymphoma with a 2.7% increase in incidence is the fifth and sixth most common cancer among females and males, respectively (The Leukemia & Lymphoma Society). In recent years, progress has been made toward understanding the molecular and cellular biology of non-Hodgkin lymphomas. Many important contributions have been made by the identification of several key genetic factors associated with non-Hodgkin lymphomas. However, the treatments of non-Hodgkin lymphomas still mainly depend on chemotherapy and radiotherapy because the molecular mechanisms underlying the pathogenesis of non-Hodgkin lymphomas remain largely unclear.

It has been established that the damage or mutation of DNA in a lymphocyte may result in the uncontrolled and excessive growth of the lymphocyte (The Leukemia & Lymphoma Society). Therefore, future strategies for the prevention and treatment of cancers will be focused on the elucidation of damaged/mutated genes, in particular, the genes located on chromosome Xp22 since this segment has been shown to be associated with the development of lymphoma. Furthermore, a correlation was reported between T-cell lymphomas and hypomagnesemia & hypocalcemia. Hypomagnesemia with secondary hypocalcemia was suggested to be caused by the disruption of a magnesium-dependent hypocalcaemia gene in chromosome Xp22. Therefore, future strategies for the prevention and treatment of T-cell lymphoma will be focused on the elucidation of the abnormal genetic substrates localized on chromosome Xp22. It is interesting to note that a human protein phosphatase with EF-hands-1 (PPEF-1) gene mapped on this region (Brunner et al. (1999) Genome Res. 9:437-48) contains EF-hand motifs at its carboxy terminus (Sherman et al., (1997) Proc Natl Acad Sci USA. 94:11639-44). The EF-hands have been shown to be involved in calcium binding (Ramulu and Nathans (2001) J Biol Chem. 276:25127-35) which strongly suggests that PPEF-1 may play a role in the development of T-cell lymphoma. Thus, the discovery of gene variants for PPEF-1 may be an important goal for the development of diagnostic markers of T-cell lymphoma.

### SUMMARY OF THE INVENTION

The present invention provides one PPEF-1-related gene variant located in human T-cell lymphoma. The nucleotide sequence of the gene variant and polypeptide sequence encoded thereby can be used for the diagnosis of any diseases associated with this gene variant or T-cell lymphoma, in particular, the T-cell lymphoblastic lymphoma.

The invention further provides an expression vector and a host cell for expressing the variant.

The invention also provides a method for producing the variant.

The invention further provides an antibody that specifically binds to the variant. For example, by selecting a sequence unique to one of the variants, e.g., a peptide fragment that spans the deletion junctions, an antibody may be generated that binds to one of the variants and not to PPEF-1. Preferably, such peptide antigens are at least 17 amino acid long. But in some cases smaller peptides such as 10mers, 12mers, or 15mers may suffice. Longer peptides, such as 20mers, 50mers, hundred-mers (and those therebetween) and even up to full length proteins may also be used.

The invention also provides methods for detecting the presence of the variant in a mammal.

### BRIEF DESCRIPTION OF THE DRAWINGS

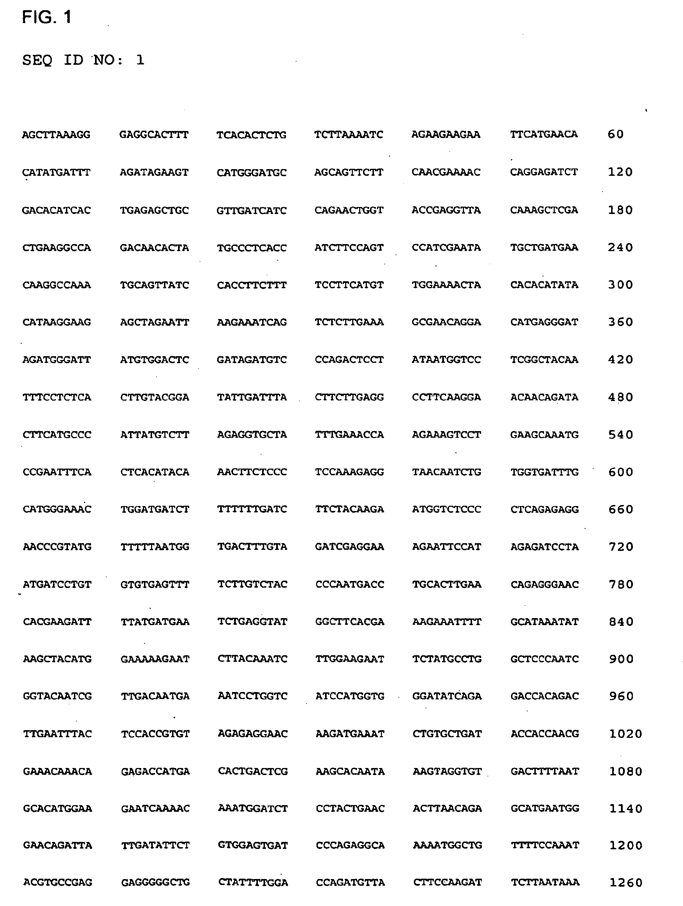

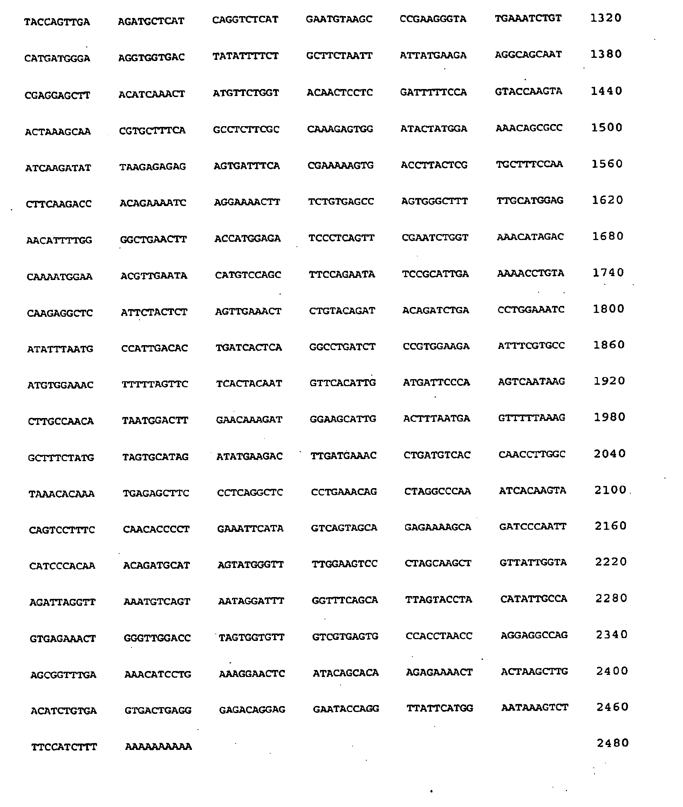

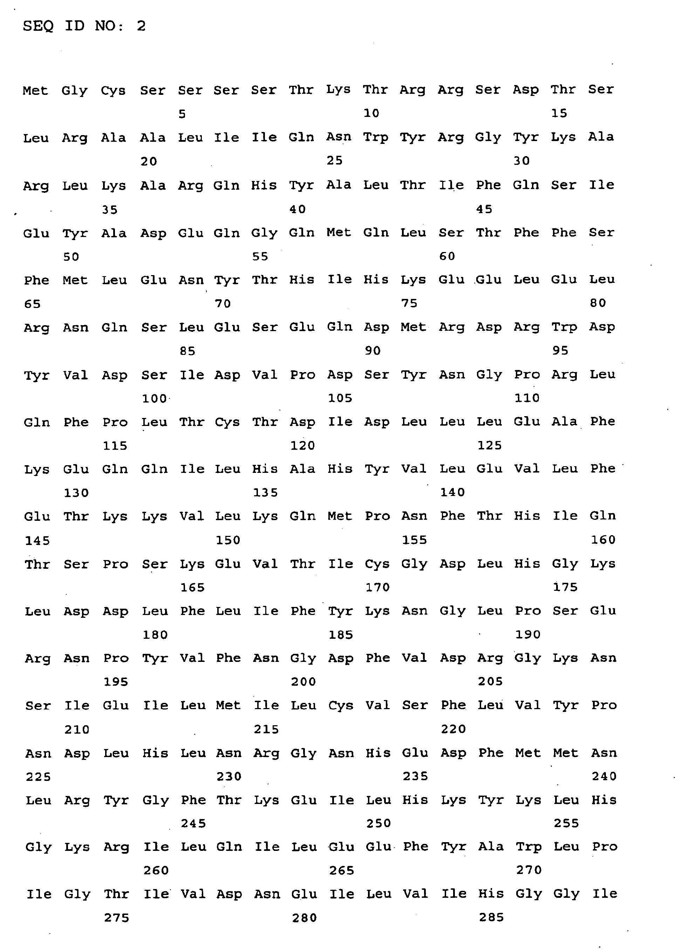

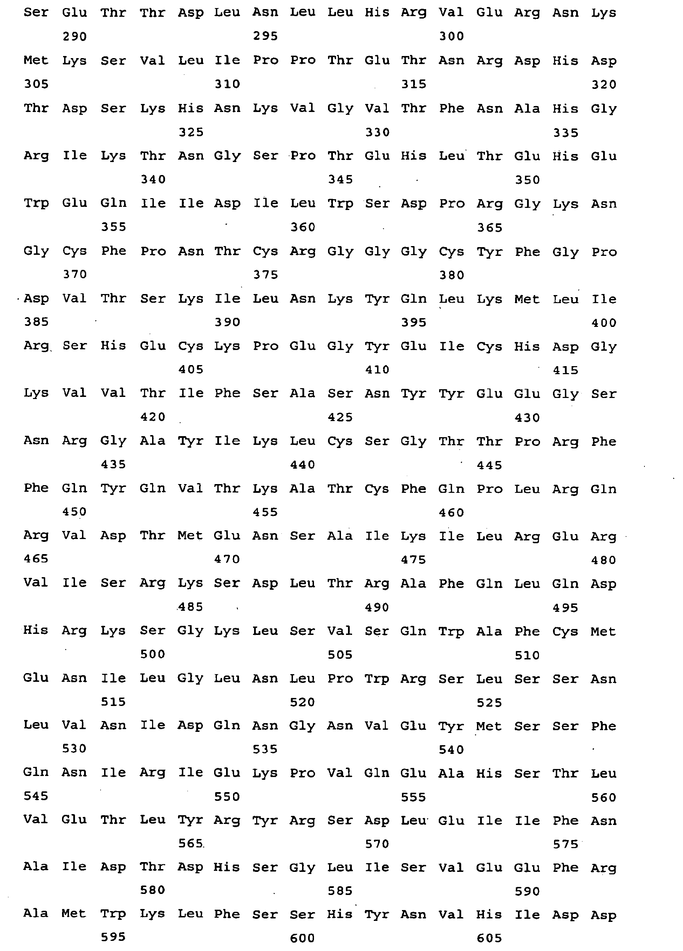

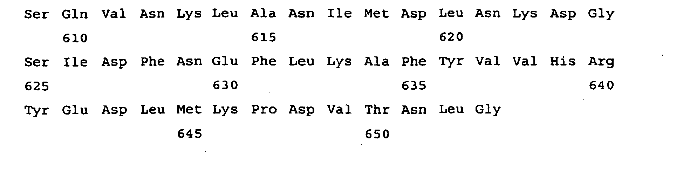

FIG. 1 shows the nucleic acid sequence (SEQ ID NO:1), amino acid sequence (SEQ ID NO:2) and nucleic acid with corresponding amino acid sequence (SEQ ID NO:1&2) of PPEF-1.

FIG. 2 shows the nucleic acid sequence (SEQ ID NO:3), amino acid sequence (SEQ ID NO:4) and nucleic acid with corresponding amino acid sequence (SEQ ID NO:3&4) of PPEF-1V.

FIG. 3 shows the nucleotide sequence alignment between the human PPEF-1 gene and its related gene variant (PPEF-1V).

FIG. 4 shows the amino acid sequence alignment between the human PPEF-1 protein and its related gene variant (PPEF-1V).

FIG. 5 shows the expression pattern of PPEF-1V in human cell lines.

### DETAILED DESCRIPTION OF THE INVENTION

All technical and scientific terms used herein, unless otherwise defined, have the same meanings as commonly understood by persons skilled in the art.

The term "antibody" used herein denotes intact molecules (a polypeptide or group of polypeptides) as well as fragments thereof, such as Fab, R(ab')₂, and Fv fragments, which are capable of binding the epitopes. Antibodies are produced by specialized B cells after stimulation by an antigen. Structurally, antibody consists of four subunits including two heavy chains and two light chains. The internal surface shape and charge distribution of the antibody binding domain is complementary to the features of an antigen. Thus, antibody can specifically act against the antigen in an immune response.

The term "base pair (bp)" used herein denotes nucleotides composed of a purine on one strand of DNA which can be hydrogen bonded to a pyrimidine on the other strand. Thymine (or uracil) and adenine residues are linked by two hydrogen bonds. Cytosine and guanine residues are linked by three hydrogen bonds.

The term "Basic Local Alignment Search Tool (BLAST; Altschul et al., (1997) Nucleic Acids Res. 25: 3389-3402)" used herein denotes programs for evaluation of homologies between a query sequence (amino or nucleic acid) and a test sequence. Specific BLAST programs are described as follows:

(1) BLASTN compares a nucleotide query sequence against a nucleotide sequence database;

(2) BLASTP compares an amino acid query sequence against a protein sequence database;

(3) BLASTX compares the six-frame conceptual translation products of a query nucleotide sequence against a protein sequence database;

(4) TBLASTN compares a query protein sequence against a nucleotide sequence database translated in all six reading frames; and

(5) TBLASTX compares the six-frame translations of a nucleotide query sequence against the six-frame translations of a nucleotide sequence database.

The term "cDNA" used herein denotes nucleic acids that synthesized from a mRNA template using reverse transcriptase.

The term "cDNA library" used herein denotes a library composed of complementary DNAs which are reverse-transcribed from mRNAs.

The term "complement" used herein denotes a polynucleotide sequence capable of forming base pairing with another polynucleotide sequence. For example, the sequence 5'-ATGGACTTACT-3' binds to the complementary sequence 5'- AGTAAGTCCAT-3'.

The term "deletion" used herein denotes a removal of a portion of one or more amino acid residues/nucleotides from a gene.

The term "expressed sequence tags (ESTs)" used herein denotes short (200 to 500 base pairs) nucleotide sequence that derives from either 5' or 3' end of a cDNA.

The term "expression vector" used herein denotes nucleic acid constructs which contain a cloning site for introducing the DNA into vector, one or more selectable markers for selecting vectors containing the DNA, an origin of replication for replicating the vector whenever the host cell divides, a terminator sequence, a polyadenylation signal, and a suitable control sequence which can effectively express the DNA in a suitable host. The suitable control sequence may include promoter, enhancer and other regulatory sequences necessary for directing polymerases to transcribe the DNA.

The term "host cell" used herein denotes a cell which is used to receive, maintain, and allow the reproduction of an expression vector comprising DNA. Host cells are transformed or transfected with suitable vectors constructed using recombinant DNA methods. The recombinant DNA introduced with the vector is replicated whenever the cell divides.

The term "insertion" or "addition" used herein denotes the addition of a portion of one or more amino acid residues/nucleotides to a gene.

The term "in silico" used herein denotes a process of using computational methods (e.g., BLAST) to analyze DNA sequences.

The term "polymerase chain reaction (PCR)" used herein denotes a method which increases the copy number of a nucleic acid sequence using a DNA polymerase and a set of primers (about 20bp oligonucleotides complementary to each strand of DNA) under suitable conditions (successive rounds of primer annealing, strand elongation, and dissociation).

The term "polynucleotide" or "nucleic acid sequence"used herein denotes a sequence of nucleotide (guanine, cytosine, thymine or adenine) in a specific order that can be a natural or synthesized fragment of DNA or RNA. It may be single-stranded or double-stranded.

The term "protein" or "polypeptide" used herein denotes a sequence of amino acids in a specific order that can be encoded by a gene or by a recombinant DNA. It can also be chemically synthesized.

The term "RNA interference (RNAi)" used herein denotes an introduction of homologous double stranded RNA (dsRNA) into a cell to specifically inhibit the expression of a gene.

The term "reverse transcriptase-polymerase chain reaction (RT-PCR)" used herein denotes a process which transcribes mRNA to complementary DNA strand using reverse transcriptase followed by polymerase chain reaction to amplify the specific fragment of DNA sequences.

The term "transformation" used herein denotes a process describing the uptake, incorporation, and expression of exogenous DNA by prokaryotic host cells.

The term "transfection" used herein denotes a process describing the uptake, incorporation, and expression of exogenous DNA by eukaryotic host cells.

The term "variant" used herein denotes a fragment of sequence (nucleotide or amino acid) inserted or deleted by one or more nucleotides/amino acids.

According to the present invention, the polypeptides of one novel human PPEF-1-related gene variant and the nucleic acid sequences encoding the same are provided.

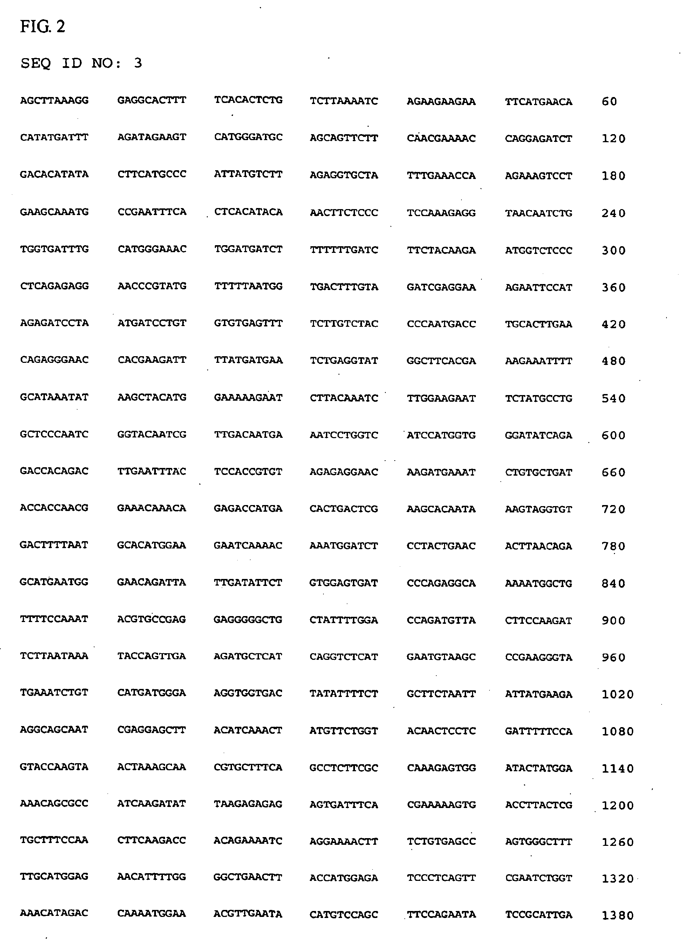

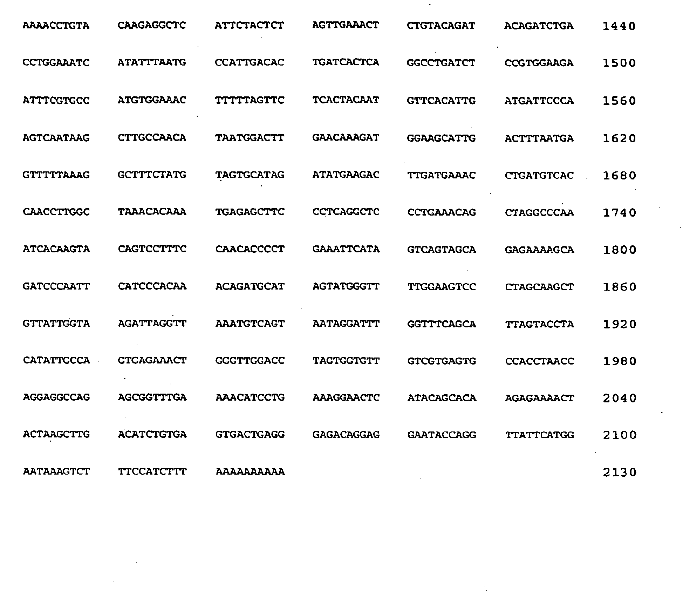

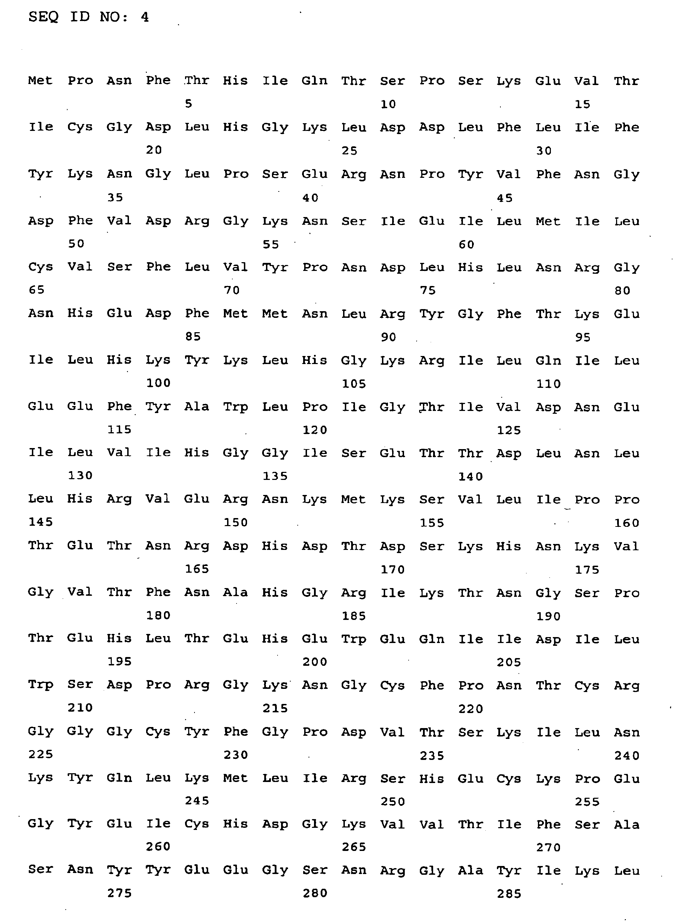

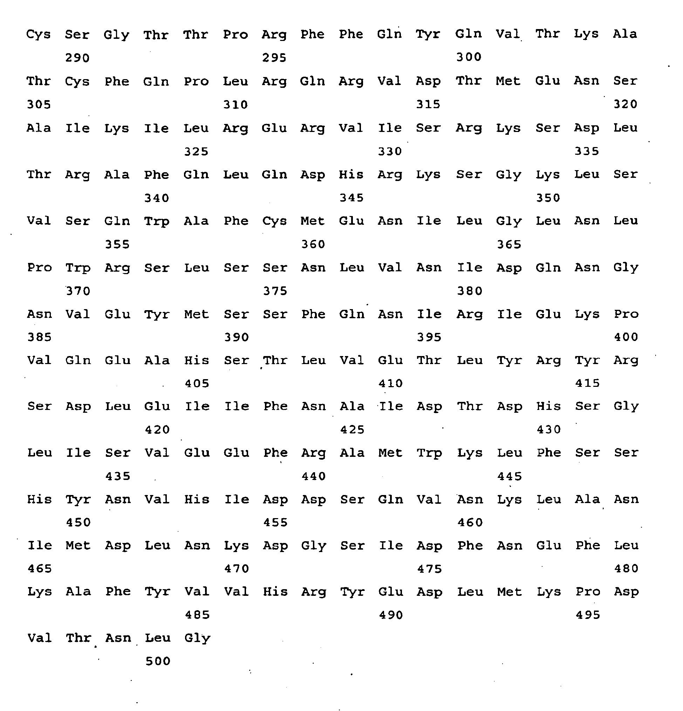

According to the present invention, human PPEF-1 cDNA sequence was used to query the human EST databases using BLAST program to search for PPEF-1-related gene variants. One human cDNA partial sequences (i.e., EST) similar to PPEF-1 was identified from ESTs deposited in a cDNA database constructed using a SUP-T1 (T-cell lymophoblastic lymphoma) cell line. The cDNA clone, named PPEF-1V (PPEF-1 variant), was then isolated from the SUP-T1 cDNA library and sequenced. FIG. 2 shows the nucleic acid sequences (SEQ ID NOs:3) of PPEF-1V and its corresponding amino acid sequences (SEQ ID NOs:4) encoded thereby.

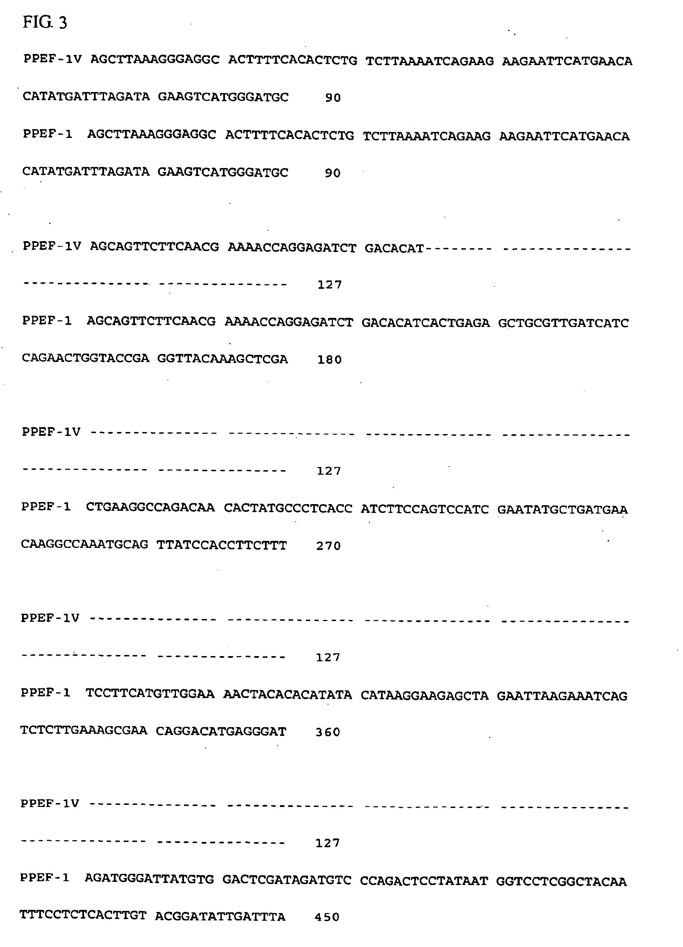

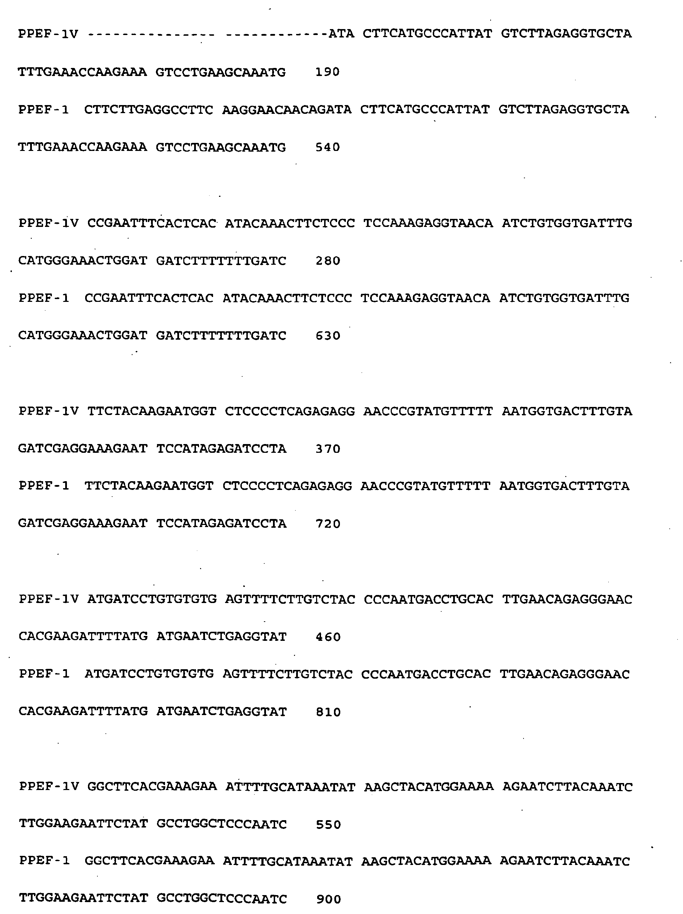

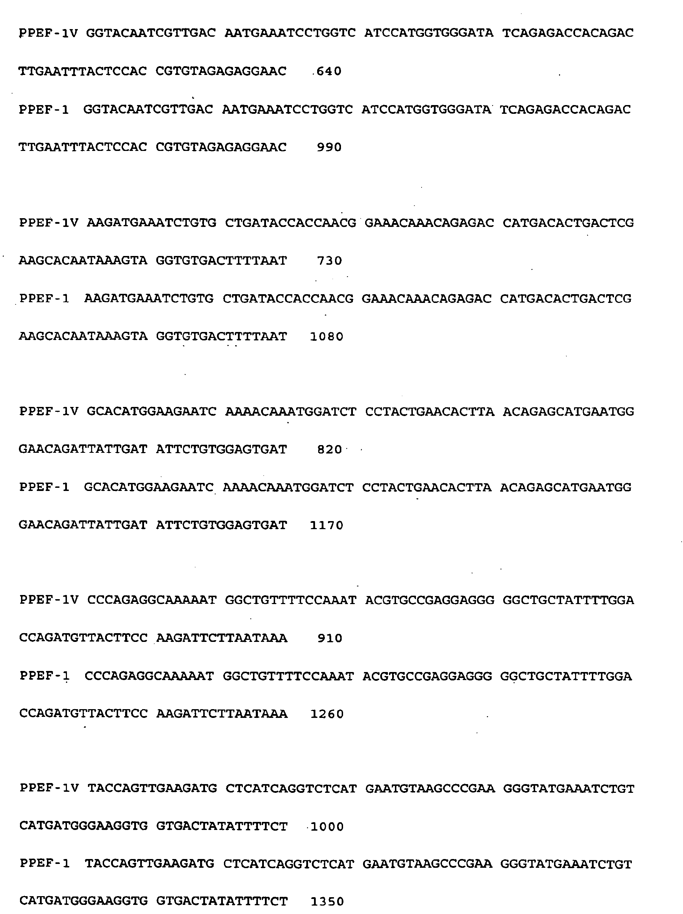

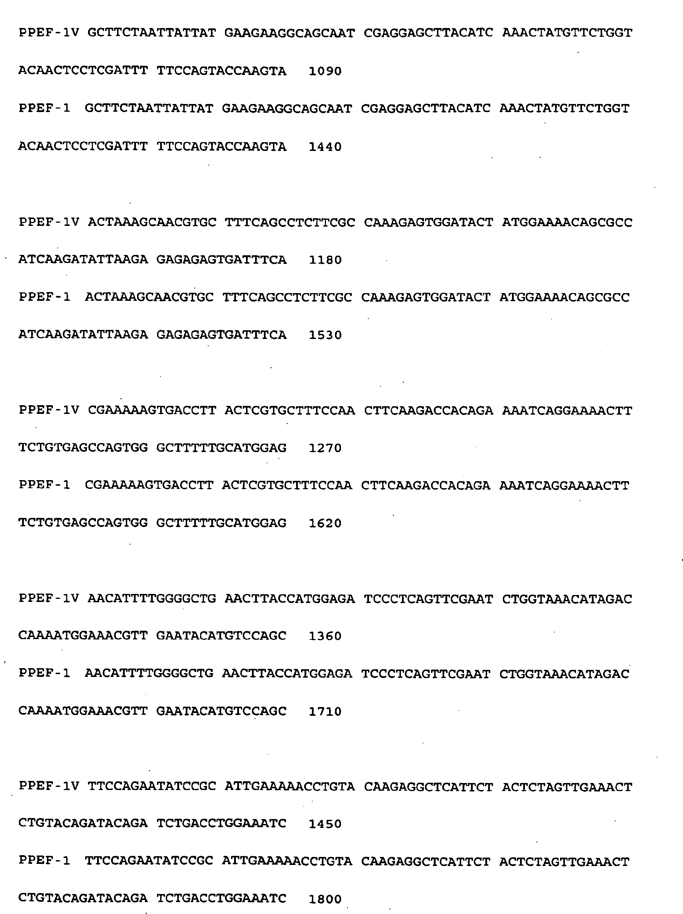

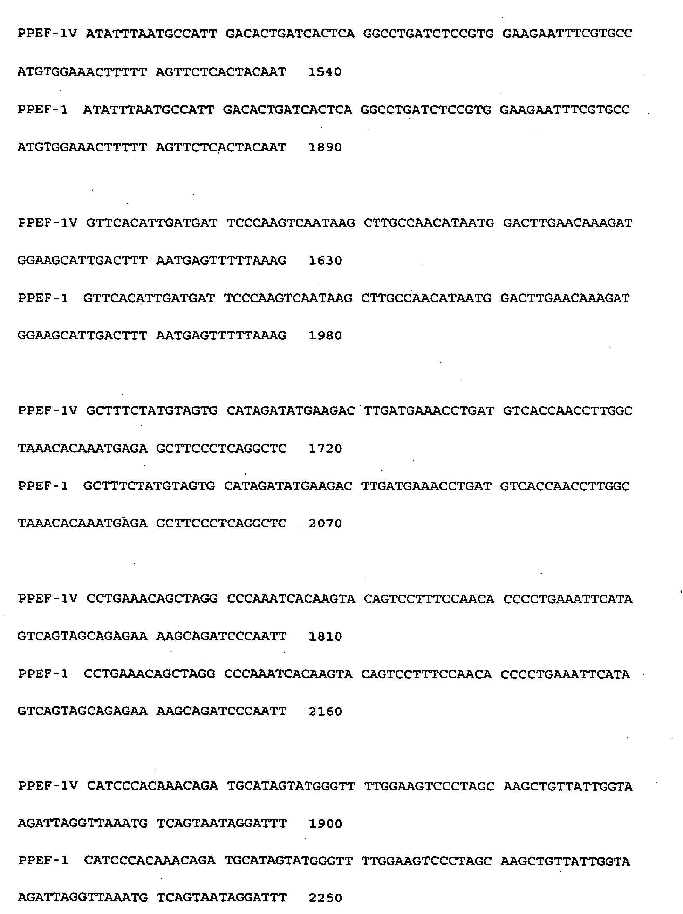

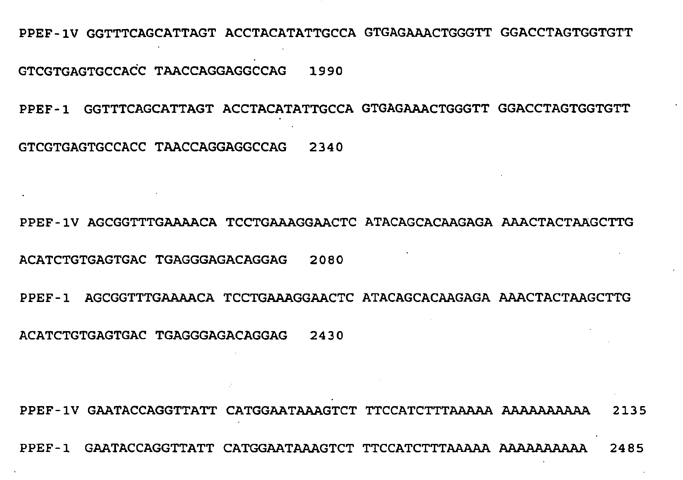

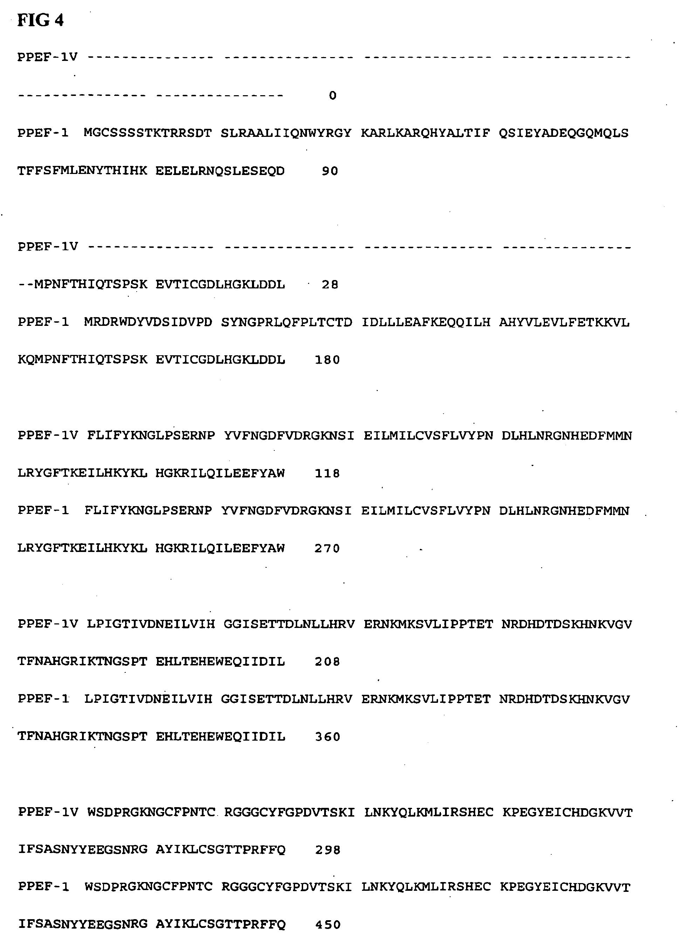

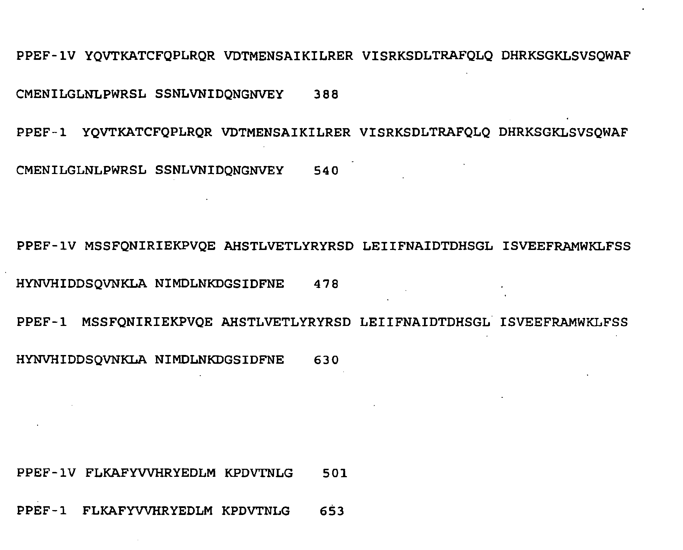

The full-length of the PPEF-1V cDNA is a 2130bp clone containing a 1503bp open reading frame (ORF) extending from nucleotides 188 to 1690, which corresponds to an encoded protein of 501 amino acid residues with a predicted molecular mass of 57.8 kDa. The initiation ATG sequence of PPEF-1V is located at nucleotides 188-190bp. To determine the variation in sequence of PPEF-1V cDNA clone, an alignment of PPEF-1 nucleotide/amino acid sequence with PPEF-1V was performed (FIGs. 3 and 4). The results indicate that one major genetic deletion was found in the aligned nucleotide sequences, indicating that PPEF-1V has a 350bp deletion in the sequence of PPEF-1 from nucleotides 128-477. Thus, the PPEF-1V protein contains a 152 amino acid N-terminal deletion comparing to the PPEF-1 protein. Scanning both PPEF-1 and PPEF-1V amino acid sequences against the profile entries in PROSITE (ScanProsite) indicated that PPEF-1 protein contains one IQ motif site (18-43aa), and three EF-hand calcium-binding domain sites (483-518aa, 566-601aa, and 606-641 aa). PPEF-1V protein contains, however, only three EF-hand calcium-binding domain sites (331-366aa, 414-449aa, and 454-489aa). It has been reported that IQ motif of PPEF-1 is a calmodulin-binding site (Kutuzov et al. (2002) Biochem Biophys Res Commun. 293:1047-52). The lacking of IQ motif in PPEF-1V suggests that the function of PPEF-1V may be independent calmodulin binding.

In the present invention, a search of ESTs deposited in dbEST at NCBI was performed to determine the presence of PPEF-1V *in silico.* The result of *in silico* analysis showed that one EST (GenBank accession number BE546038) was found to confirm the absence of 350bp region on PPEF-1V nucleotide sequence. Therefore, any nucleotide fragments comprising the immediately upstream and downstream sequences of the 350bp deleted region (128-477bp) of PPEF-1V are the "gene targets" and may be used as probes to determine the presence of PPEF-1V under high stringency conditions. An alternative approach is that any set of primers for amplifying the fragment containing the immediately upstream and downstream sequences of the 350bp deleted region (128-477bp) of PPEF-1V are the "gene targets" and may be used to determine the presence of the variant.

According to the present invention, the polypeptides of the human PPEF-1V and its fragments thereof may be produced via genetic engineering techniques. In this case, they are produced by appropriate host cells which have been transformed by DNAs that code for the polypeptides or fragments thereof. The nucleotide sequence encoding the polypeptide of the human PPEF-1V or their fragments thereof is inserted into an appropriate expression vector, i.e., a vector which contains the necessary elements for the transcription and translation of the inserted coding sequence in a suitable host. The nucleic acid sequence is inserted into the vector where the sequence will be expressed under appropriate conditions (e.g., in proper orientation and correct reading frame and with appropriate expression sequences, including an RNA polymerase binding sequence and a ribosomal binding sequence).

Any method that is known to those skilled in the art may be used to construct expression vectors containing sequences encoding the polypeptides of the human PPEF-1V and appropriate transcriptional/translational control elements. These methods may include *in vitro* recombinant DNA and synthetic techniques, and *in vivo* genetic recombinants.

A variety of expression vector/host systems may be utilized to express the polypeptide-coding sequence. These include, but not limited to, microorganisms such as bacteria transformed with recombinant bacteriophage, plasmid, or cosmid DNA expression vector; yeast transformed with yeast expression vector; insect cell systems infected with virus (e.g., baculovirus); plant cell system transformed with viral expression vector (e.g., cauliflower mosaic virus, CaMV, or tobacco mosaic virus, TMV); or animal cell system infected with virus (e.g., vaccina virus, adenovirus, etc.). Preferably, the host cell is a bacterium, and most preferably, the bacterium is *E. coli.*

Alternatively, the Polypeptides of the human PPEF-1V or fragments thereof may be synthesized using chemical methods. For example, peptide synthesis can be performed using various solid-phase techniques. Automated synthesis may be achieved using the ABI 431A peptide synthesizer (Perkin-Elmer).

According to the present invention, the fragments of the polypeptides and nucleic acid sequences of the human PPEF-1V are used as immunogens, primers or probes. Preferably, the purified fragments of the human PPEF-1V are used. The fragments may be produced by enzyme digestion, chemical cleavage of isolated or purified polypeptide or nucleic acid sequences, or chemical synthesis. The fragment then may be isolated or purified. Such isolated or purified fragments of the polypeptides and nucleic acid sequences can be used as immunogens, primers or probes.

The present invention further provides the antibodies which specifically bind one or more out-surface epitopes of the polypeptides of the human PPEF-1V.

According to the present invention, immunization of mammals such as humans, rabbits, rats, mice, sheep, goats, cows, or horses with immunogens described herin is performed using procedures well known to those skilled in the art, for the purpose of obtaining antisera containing polyclonal antibodies or hybridoma lines secreting monoclonal antibodies.

Monoclonal antibodies can be prepared by standard techniques, given the teachings contained herein. Such techniques are disclosed, for example, in U.S. patent number 4,271,145 and U.S. patent number 4,196,265. Briefly, an animal is immunized with the immunogen. Hybridomas are prepared by fusing spleen cells from the immunized animal with myeloma cells. The fused products are screened for those producing antibodies that specifically bind to the immunogen. The positive hybridoma clones are isolated, and the monoclonal antibodies are recovered from those clones.

Immunization regimens for production of both polyclonal and monoclonal antibodies are well-known in the art. The immunogen may be injected by any route, including subcutaneous, intravenous, intraperitoneal, intradermal, intramuscular, mucosal, or a combination thereof. The immunogen may be injected in soluble form, aggregate form, attached to a physical carrier, or mixed with an adjuvant. The antisera and antibodies may be purified using column chromatography methods.

According to the present invention, antibody fragments which contain specific binding sites for the polypeptides or fragments thereof may also be generated. For example, such fragments include, but are not limited to, F(ab')₂ fragments made by pepsin digestion of the antibody molecule or Fab fragments generated by reducing the disulfide bridges of the F(ab')₂ fragments.

The subject invention also provides methods for diagnosing the diseases associated with PPEF-1V or T-cell lymphoma, more preferably, the T-cell lymphoblastic lymphoma, by utilizing the nucleic acid sequences, the polypeptide of the human PPEF-1V, or fragments thereof, and the antibodies against the polypeptides.

Many gene variants have been found to associate with diseases (Stallings-Mann et al., (1996) Proc Natl Acad Sci U S A 93: 12394-9; Liu et al., (1997) Nat Genet 16:328-9; Siffert et al., (1998) Nat Genet 18: 45 to 8; Lukas et al., (2001) Cancer Res 61: 3212 to 9). Since PPEF-1V clone was isolated from T-cell lymphoblastic lymphoma cell line (SUP-T1), it is advisable that PPEF-1V serves as a marker for the diagnosis of human T-cell lymphoblastic lymphoma. Thus, the expression level of PPEF-1V relative to PPEF-1 may be a useful indicator to screen patients suspected of having T-cell lymphoma or more specifically the T-cell lymphoblastic lymphoma. This suggests that the index of relative expression level (mRNA or protein) may confer an increased susceptibility to T-cell lymphoma, more preferably, the T-cell lymphoblastic lymphoma. Fragments of PPEF-1V transcripts (mRNAs) may be detected by RT-PCR approach. Polypeptides of PPEF-1V may be determined by the binding of antibodies to these polypeptides. On the other hand, a method (RNAi; RNA interference) that is known to those skilled in the art may be used to interfere and degrade the targeted RNA using chemically synthesized double stranded short nucleic acid (about 23 nucleotides dsRNA) molecules (Montgomery et al. (1998) Proc Natl Acad Sci U S A. 95:15502-7). According to the present invention, the double stranded short nucleic acid molecules containing the immediately upstream and downstream sequences of the 350bp deleted region (128-477bp) of PPEF-1V may be used to interfere and degrade PPEF-1V mRNA. After RNAi approach was conducted, the decreased transcripts or polypeptides may be used to determine the presence of PPEF-1V mRNA.

According to the present invention, the expression of these gene variant mRNAs may be determined by, but not limited to, RT-PCR. Using TRIZOL reagents (Life Technology), total RNA may be isolated from patient samples. Tissue samples (e.g., biopsy samples) are powdered under liquid nitrogen before homogenization. RNA purity and integrity are assessed by absorbance at 260/280 nm and by agarose gel electrophoresis. A set of primers can be designed to amplify the expected size of specific PCR fragments of PPEF-1V. For example, one of the primers is a fragment of the sequences (A fragment) or a fragment complementary to the sequences (B fragment) containing the gene targets: nucleotides 127-128 of PPEF-1V; the other is a fragment of the PPEF-1V sequences designed at any location upstream of "B fragment" or a fragment complementary to the PPEF-1V sequences designed at any location downstream of "A fragment". Alternatively, one primer is designed at any location upstream of nucleotide 127 of PPEF-1V and the other is designed at any location complementary to a portion downstream of nucleotide 128 of PPEF-1V. The length of the PCR fragment from PPEF-1V will be 350bp shorter than that from PPEF-1. PCR fragments are analyzed on a 1% agarose gel using five microliters (10%) of the amplified products. The intensity of the signals may be determined by using the Molecular Analyst program (version 1.4.1; Bio-Rad). Thus, the index of relative expression levels for each co-amplified PCR products may be calculated based on the signal intensities.

The RT-PCR experiment may be performed according to the manufacturer instructions (Boehringer Mannheim). A 50µl reaction mixture containing 2µl total RNA (0.1µg/µl), 1µl each primer (20 pM), 1µl each dNTP (10 mM), 2.5 µl DTT solution (100 mM), 10 µl 5X RT-PCR buffer, 1µl enzyme mixture, and 28.5 µl sterile distilled water may be subjected to the conditions such as reverse transcription at 60°C for 30 minutes followed by 35 cycles of denaturation at 94°C for 2 minutes, annealing at 60°C for 2 minutes, and extension at 68°C for 2 minutes. The RT-PCR analysis may be repeated twice to ensure reproducibility, for a total of three independent experiments.

The expression of gene variants can also be analyzed using Northern Blot hybridization approach. Specific fragment of the PPEF-1V may be amplified by polymerase chain reaction (PCR) using primer set designed for RT-PCR. The amplified PCR fragment may be labeled and served as a probe to hybridize the membranes containing total RNAs extracted from the samples under the conditions of 55°C in a suitable hybridization solution for 3 hours. Blots may be washed twice in 2 x SSC, 0.1% SDS at room temperature for 15 minutes each, followed by two washes in 0.1 x SSC and 0.1% SDS at 65°C for 20 minutes each. After these washes, blot may be rinsed briefly in suitable washing buffer and incubated in blocking solution for 30 minutes. Then, the blot may be incubated in suitable antibody solution for 30 minutes. Blots may be washed in washing buffer for 30 minutes and equilibrated in suitable detection buffer before detecting the signals. Alternatively, the presence of gene variants (cDNAs or PCR) can be detected using microarray approach. The cDNAs or PCR products corresponding to the nucleotide sequences of the present invention may be immobilized on a suitable substrate such as a glass slide. Hybridization can be preformed using the labeled mRNAs extracted from samples. After hybridization, nonhybridized mRNAs are removed. The relative abundance of each labeled transcript, hybridizing to a cDNA/PCR product immobilized on the microarray, can be determined by analyzing the scanned images.

According to the present invention, the presence of the polypeptide of PPEF-1V may be determined by, but not limited to, the immunoassay which uses the antibody that specifically binds to the polypeptide. The polypeptides of the gene variants may be expressed in prokaryotic cells by using suitable prokaryotic expression vectors. The cDNA fragments of PPEF-1V gene may be PCR amplified using primer set A with restriction enzyme digestion sites incorporated in the 5' and 3' ends, respectively. The PCR products can then be enzyme digested, purified, and inserted into the corresponding sites of prokaryotic expression vector in-frame to generate recombinant plasmids. Sequence fidelity of this recombinant DNA can be verified by sequencing. The prokaryotic recombinant plasmids may be transformed into host cells (e.g., *E. coli* BL21 (DE3)). Recombinant protein synthesis may be stimulated by the addition of 0.4 mM isopropylthiogalactoside (IPTG) for 3 hours. The bacterially-expressed proteins may be purified.

The polypeptide of the gene variant may be expressed in animal cells by using eukaryotic expression vectors. Cells may be maintained in Dulbecco's modified Eagle's medium (DMEM) supplemented with 10% fetal bovine serum (FBS; Gibco BRL) at 37°C in a humidified 5% CO₂ atmosphere. Before transfection, the nucleotide sequence of each of the gene variant may be amplified with PCR primers containing restriction enzyme digestion sites and ligated into the corresponding sites of eukaryotic expression vector in-frame. Sequence fidelity of this recombinant DNA can be verified by sequencing. The cells may be plated in 12-well plates one day before transfection at a density of 5 x 10⁴ cells per well. Transfections may be carried out using Lipofectaminutese Plus transfection reagent according to the manufacturer's instructions (Gibco BRL). Three hours following transfection, the medium containing the complexes may be replaced with fresh medium. Forty-eight hours after incubation, the cells may be scraped into lysis buffer (0.1 M Tris HCl, pH 8.0, 0.1% Triton X-100) for purification of expressed proteins. After these proteins are purified, monoclonal antibodies against these purified proteins (PPEF-1V) may be generated using hybridoma technique according to the conventional methods (de StGroth and Scheidegger, (1980) J Immunol Methods 35:1-21; Cote et al. (1983) Proc Natl Acad Sci U S A 80: 2026-30; and Kozbor et al. (1985) J Immunol Methods 81:31-42).

According to the present invention, the presence of the polypeptides of PPEF-1 V in samples of T-cell lymphoblastic lymphoma may be determined by, but not limited to, Western blot analysis. Proteins extracted from samples may be separated by SDS-PAGE and transferred to suitable membranes such as polyvinylidene difluoride (PVDF) in transfer buffer (25 mM Tris-HCl, pH 8.3, 192 mM glycine, 20% methanol) with a Trans-Blot apparatus for 1 hour at 100 V (e.g., Bio-Rad). The proteins can be immunoblotted with specific antibodies. For example, membrane blotted with extracted proteins may be blocked with suitable buffers such as 3% solution of BSA or 3% solution of nonfat milk powder in TBST buffer (10 mM Tris-HCl, pH 8.0, 150 mM NaCl, 0.1 % Tween 20) and incubated with monoclonal antibody directed against the polypeptides of gene variants. Unbound antibody is removed by washing with TBST for 5 X 1 minutes. Bound antibody may be detected using commercial ECL Western blotting detecting reagents.

The following examples are provided for illustration, but not for limiting the invention.

### EXAMPLES

### Analysis of Human T-cell lymphoblastic lymphoma EST Database

Expressed sequence tags (ESTs) generated from the large-scale PCR-based sequencing of the 5'-end of human T-cell lymphoblastic lymphoma cDNA clones were compiled and served as EST databases. Sequence comparisons against the nonredundant nucleotide and protein databases were performed using BLASTN and BLASTX programs, at the National Center for Biotechnology Information (NCBI) with a significance cutoff of p<10⁻¹⁰. ESTs representing putative PPEF-1V gene were identified during the course of EST generation.

### Isolation of cDNA Clones

One cDNA clone exhibiting EST sequence similar to the PPEF-1 gene was isolated from the T-cell lymphoblastic lymphoma cDNA library and named PPEF-1V. The inserts of these clones were subsequently excised *in vivo* from the λZAP Express vector using the ExAssist/XLOLR helper phage system (Stratagene). Phagemid particles were excised by coinfecting XL1-BLUE MRF' cells with ExAssist helper phage. The excised pBluescript phagemids were used to infect *E. coli* XLOLR cells, which lack the amber suppressor necessary for ExAssist phage replication. Infected XLOLR cells were selected using kanamycin resistance. Resultant colonies contained the double stranded phagemid vector with the cloned cDNA insert. A single colony was grown overnight in LB-kanamycin, and DNA was purified using a Qiagen plasmid purification kit.

### Full Length Nucleotide Sequencing and Database Comparisons

Phagemid DNA was sequenced using the *Taq* dye-deoxy terminator cycle sequencing kit for the Applied Biosystems 377 sequencing system (PerkinElmer Life Sciences). Using the primer-walking approach, full-length sequence was determined. Nucleotide and protein searches were performed using BLAST against the non-redundant database of NCBI.

### In Silico Sequence Similarity Analysis

The coding sequence for each cDNA clones was searched against the dbEST sequence database using the BLAST algorithm at the NCBI website. ESTs derived from each tissue were used as a source of information for transcript tissue expression analysis. Tissue distribution for each isolated cDNA clone was determined by ESTs matching to that particular sequence variants (insertions or deletions) with a significance cutoff of p<10⁻¹⁰.

### RT-PCR Expression Pattern Analysis

The expression pattern of PPEF-1V was conducted in human cell lines using RT-PCR. The human cell lines were WI38 (Lung, fetus); A549 (Lung adenocarcinoma); H661 (Large cell carcinoma, lung); H520 (Squamous cell carcinoma, lung); H209 (Small cell carcinoma, lung); JHH-4 (Hepatoma); SUP-T1 (T-cell lymophoblastic lymphoma); Daudi (Burkitt's lymophoma); Ramos (Burkitt's lymophoma); RAJI (Burkitt's lymophoma); ZR75-1 (Breast carcinoma); MDA-MB-231 (Breast adenocarcinoma); Hs 578T (Breast carcinoma); G5T/VGH (Glioblastoma multiforme); TSGH9201 (Gastric carcinoma); Ca Ski (Cervix epidermoid carcinoma); Hela S3 (Cervical epitheloid carcinoma); COLO 320 HSR (Colon adenocarcinoma); SW620 (Adenocarcinoma); TSGH8301 (Urinary bladder carcinoma); ES-2 (Ovarian carcinoma); DU145 (Brain prostate carcinoma); MIA paca-2 (Pancreatic carcinoma); CE48T/VGH (Esophagus epidermoid carcinoma); CE81T/VGH (Esophagus carcinoma well differentiated aquamous); HL-CZ (Promonocytic leukemia); Hs 181.tes (Normal testis). The purity and integrity of total RNA extracted from each of the cell lines were assessed by absorbance at 260/280 nm and by agarose gel electrophoresis. The forward and reverse primers for PPEF-1V were: 5'-CTGACACATATACTTCATGCCC-3' and 5'-CACACAGGATCATTAGGATCTC-3'. The expected sizes of PPEF-1V PCR fragments was 265bp.

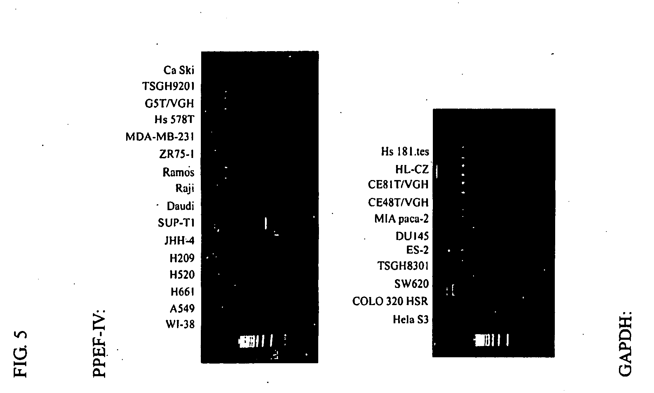

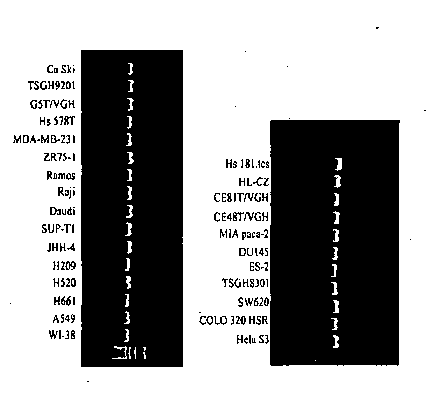

Glyceraldehyde-3-phosphate dehydrogenase (GAPDH; accession No. M33197) was used for internal control. The forward and reverse primers for GAPDH were: 5'-TGGGTGTGAACCATGAGAAG-3' and 5'-GTGTCGCTGTTGAAGTCAGA-3'. The expected sizes of GAPDH PCR fragments was 472 bp. Briefly, a 50 µl reaction mixture containing 2µl total RNA (0.1µg/µl), 1µl each primer (20 pM), 1µl each dNTP (10 mM), 2.5 µl DTT solution (100 mM), 10 µl 5X RT-PCR buffer, 1µl enzyme mixture and 28.5 µl sterile redistilled water were subjected to reverse transcription at 60°C for 30 minutes followed by 35 cycles of denaturation at 94°C for 2 minutes, annealing at 60°C for 2 minutes, and extension at 68°C for 2 minutes. Five microliters (10%) of the amplified products mixed with 1 µl of loading buffer were separated on a 1% horizontal agarose gel stained with ethidium bromide in 0.5X TAE buffer. The gel was electrophoresed at 100 V for 45 minutes. Figure 5 showed that PPEF-1V mRNA was only highly expressed in the cell line of T-cell lymophoblastic lymphoma suggesting that PPEF-1V could be used for diagnosing T-cell lymphoma, in particular, T-cell lymophoblastic lymphoma. Shown on the left is 100 bp DNA ladder markers.

### REFERENCES

All references are listed herein for the convenience of the reader.
Brunner et al. Genomic structure and comparative analysis of nine Fugu genes: conservation of synteny with human chromosome Xp22.2-p22.1. Genome Res. 9:437-48, (1999).
Ramulu and Nathans, Cellular and subcellular localization, N-terminal acylation, and calcium binding of Caenorhabditis elegans protein phosphatase with EF-hands. J Biol Chem. 276:25127-35, (2001)
Sherman et al. Identification and characterization of a conserved family of protein serine/threonine phosphatases homologous to Drosophila retinal degeneration C. Proc Natl Acad Sci U S A. 94:11639-44, (1997).

## Claims

1. An isolated polypeptide comprising an N-terminal deletion variant of the PPEF-1 protein comprising the amino acid sequence of SEQ ID NO: 4, wherein at the N-terminus of the amino acid sequence of SEQ ID NO: 4 no further protein sequence is present.

2. An isolated nucleic acid encoding said polypeptide of Claim 1.

3. The isolated nucleic acid of Claim 2, comprising the nucleotide sequence of SEQ ID NO: 3.

4. An isolated nucleic acid comprising at least 30 consecutive nucleotides selected from SEQ ID NO: 3 and that includes a di-nucleotide of nucleotides 127 to 128 of SEQ ID NO: 3.

5. An expression vector comprising the nucleic acid of any one of Claims 2 to 4.

6. A host cell comprising said expression vector of Claim 5.

7. A method for producing the polypeptide of Claim 1, which comprises the steps of:
(1) culturing the host cell of Claim 6 under a condition suitable for the expression of a PPEF variant polypeptide; and
(2) recovering said PPEF variant polypeptide from the host cell culture.

8. An antibody that specifically binds to said polypeptide of Claim 1.

9. The antibody of Claim 8, which is a polyclonal or monoclonal antibody.

10. A method for detecting the presence of a PPEF nucleic acid in a mammal, which comprises the steps of:
(1) extracting total RNA from a sample obtained from said mammal;
(2) amplifying said RNA by reverse transcriptase-polymerase chain reaction (RT-PCR) to obtain a cDNA sample;
(3) hybridizing said cDNA sample with the nucleic acid of any one of Claims 2 to 4; and
(4) detecting said hybridization.

11. The method of Claim 10, wherein said hybridizing process is conducted by Northern blot or microarray to diagnose a plurality of cancers.

12. The method of Claim 11, wherein said plurality of cancers include T-cell lymphoma.

13. The method of Claim 12, wherein said T-cell lymphoma is T-cell lymophoblastic lymphoma.

14. A method for detecting the presence of a PPEF polypeptide or fragment thereof in a mammal, which comprises the steps of:
(a) contacting proteins obtained from a sample of a mammal with an antibody that specifically binds to a PPEF polypeptide or a fragment thereof of SEQ ID NO: 4 but not to SEQ ID NO:2; and
(b) detecting said antibody-PPEF antigen binding.

15. The method of Claim 14, wherein said antibody-antigen binding samples are detected by Western blot approach to help diagnose a plurality of cancers.

16. The method of Claim 15, wherein said plurality of cancers include T-cell lymphoma.

17. The method of Claim 16, wherein said T-cell lymphoma is T-cell lymophoblastic lymphoma.

## Patentansprüche

1. Isoliertes Polypeptid, umfassend eine N-terminale Deletionsvariante des PPEF-1-Proteins, umfassend die Aminosäuresequenz der SEQ ID NO: 4, wobei am N-Terminus der Aminosäuresequenz der SEQ ID NO: 4 keine weitere Proteinsequenz vorliegt.

2. Isolierte Nukleinsäure, codierend für das Polypeptid nach Anspruch 1.

3. Isolierte Nukleinsäure nach Anspruch 2, umfassend die Nukleotidsequenz der SEQ ID NO: 3.

4. Isolierte Nukleinsäure, umfassend wenigstens 30 aufeinanderfolgende Nukleotide, ausgewählt aus SEQ ID NO: 3, wobei die Nukleinsäure ein Dinukleotid aus Nukleotiden 127 bis 128 der SEQ ID NO: 3 enthält.

5. Expressionsvektor, umfassend die Nukleinsäure nach einem der Ansprüche 2 bis 4.

6. Wirtszelle, umfassend den Expressionsvektor nach Anspruch 5.

7. Verfahren zur Herstellung des Polypeptids nach Anspruch 1, das folgende Schritte umfasst:
(1) Kultivieren der Wirtszelle nach Anspruch 6 unter einer für die Expression einer PPEF-Polypeptidvariante geeigneten Bedingung und
(2) Isolieren der PPEF-Polypeptidvariante aus der Wirtszellkultur.

8. Antikörper, der spezifisch an das Polypeptid nach Anspruch 1 bindet.

9. Antikörper nach Anspruch 8, bei dem es sich um einen polyklonalen oder monoklonalen Antikörper handelt.

10. Verfahren zum Nachweis des Vorliegens einer PPEF-Nukleinsäure in einem Säuger, das folgende Schritte umfasst:
(1) Extrahieren von Gesamt-RNA aus einer dem Säuger entnommenen Probe;
(2) Amplifizieren der RNA mittels Reverse-Transkriptase-Polymerasekettenreaktion (RT-PCR) zur Gewinnung einer cDNA-Probe;
(3) Hybridisieren der cDNA-Probe mit der Nukleinsäure nach einem der Ansprüche 2 bis 4; und
(4) Nachweisen der Hybridisierung.

11. Verfahren nach Anspruch 10, wobei der Hybridisierungsvorgang mittels Northern-Blot oder Mikroarray zur Diagnose mehrerer Krebserkrankungen ausgeführt wird.

12. Verfahren nach Anspruch 11, wobei die mehreren Krebserkrankungen T-Zellen-Lymphom einschließen.

13. Verfahren nach Anspruch 12, wobei es sich bei dem T-Zellen-Lymphom um T-lymphoblastisches Lymphom handelt.

14. Verfahren zum Nachweis des Vorliegens eines PPEF-Polypeptids oder eines Fragments davon in einem Säuger, das folgende Schritte umfasst:
(1) Kontaktieren von aus einer Probe eines Säugers entnommenen Proteinen mit einem Antikörper, der an ein PPEF-Polypeptid oder ein Fragment davon der SEQ ID NO: 4, jedoch nicht an die SEQ ID NO: 2, spezifisch bindet; und
(2) Nachweisen der Antikörper-PPEF-Antigen-Bindung.

15. Verfahren nach Anspruch 14, wobei die Antikörper-Antigen-Bindungsproben mit einem Western-Blot-Ansatz nachgewiesen werden als Hilfe zur Diagnose mehrerer Krebserkrankungen.

16. Verfahren nach Anspruch 15, wobei die mehreren Krebserkrankungen T-Zellen-Lymphom einschließen.

17. Verfahren nach Anspruch 16, wobei es sich bei dem T-Zellen-Lymphom um T-lymphoblastisches Lymphom handelt.

## Revendications

1. Polypeptide isolé comprenant un variant par délétion en extrémité N de la protéine PPEF-1 comprenant la séquence d'acides aminés de SEQ ID n° : 4, dans lequel à l'extrémité N de la séquence d'acides aminés de SEQ ID n° : 4 aucune autre séquence de protéine n'est présente.

2. Acide nucléique isolé codant pour ledit polypeptide de la revendication 1.

3. Acide nucléique isolé de la revendication 2, comprenant la séquence nucléotidique de SEQ ID n° : 3.

4. Acide nucléique isolé comprenant au moins 30 nucléotides consécutifs choisis à partir de SEQ ID n° : 3 et qui comprend un dinucléotide constitué par les nucléotides 127 à 128 de SEQ ID n° : 3.

5. Vecteur d'expression comprenant l'acide nucléique de l'une quelconque des revendications 2 à 4.

6. Cellule hôte comprenant ledit vecteur d'expression de la revendication 5.

7. Procédé pour produire le polypeptide de la revendication 1, lequel comprend les étapes suivantes:
(1) effectuer la culture de la cellule hôte de la revendication 6 dans des conditions appropriées pour l'expression d'un polypeptide variant de PPEF ; et
(2) récupérer ledit polypeptide variant de PPEF à partir de la culture de cellule hôte.

8. Anticorps qui se lie spécifiquement audit polypeptide de la revendication 1.

9. Anticorps de la revendication 8, lequel est un anticorps polyclonal ou monoclonal.

10. Procédé pour détecter la présence d'un acide nucléique de PPEF chez un mammifère, lequel comprend les étapes suivantes:
(1) extraire l'ARN total d'un échantillon obtenu auprès dudit mammifère ;
(2) amplifier ledit ARN par transcription inverse-amplification en chaîne par polymérase (RT-PCR) pour obtenir un échantillon d'ADNc ;
(3) hybrider ledit échantillon d'ADNc avec l'acide nucléique de l'une quelconque des revendications 2 à 4 ; et
(4) détecter ladite hybridation.

11. Procédé de la revendication 10, dans lequel ledit procédé d'hybridation est effectué par transfert Northern ou puce à ADN pour diagnostiquer une pluralité de cancers.

12. Procédé de la revendication 11, dans lequel ladite pluralité de cancers comprend le lymphome T.

13. Procédé de la revendication 12, dans lequel ledit lymphome T est un lymphome lymphoblastique T.

14. Procédé pour détecter la présence d'un polypeptide PPEF ou d'un fragment de celui-ci chez un mammifère, lequel comprend les étapes suivantes:
(a) mettre en contact des protéines obtenues à partir d'un échantillon d'un mammifère avec un anticorps qui se lie spécifiquement à un polypeptide PPEF ou un fragment de celui-ci de SEQ ID n° : 4 mais pas à SEQ ID n° : 2 ; et
(b) détecter ladite liaison anticorps-antigène PPEF.

15. Procédé de la revendication 14, dans lequel lesdits échantillons à liaison anticorps-antigène sont détectés par une approche de transfert Western pour aider au diagnostic d'une pluralité de cancers.

16. Procédé de la revendication 15, dans lequel ladite pluralité de cancers comprend le lymphome T.

17. Procédé de la revendication 16, dans lequel ledit lymphome T est un lymphome lymphoblastique T.
